# EUROPEAN PATENT APPLICATION

(11) **EP 2 151 779 A2**
(43) Date of publication of application: **10.02.2010**
(21) Application number: 09166557.0
(22) Date of filing: 28.07.2009
(51) Int. Cl.: G06F 19/00

(54) **Ultrasound system and method of offering preview pages**

(30) Priority: 31.07.2008 KR 20080074970
(71) Applicant: MEDISON CO., LTD., Gangwon-do 250-875 (KR)
(72) Inventor: Lee, Yun Hee, 135-851, Seoul (KR)
(74) Representative: Schmid, Wolfgang

(57) **Abstract**

An ultrasound system and a method of offering preview pages on a screen are disclosed. The ultrasound system of the present invention stores a plurality of report pages including information of at least one target object, forms a plurality of preview pages of the report pages and controls display of the preview pages and the report pages, wherein the preview pages and the report pages correspond one to one. A report page and the preview pages are displayed on the screen at a time. In response to a preview page selection among the preview pages displayed on the screen, the report page displayed on the screen is replaced with the report page corresponding to the preview page selection.

## Description

The present application claims priority from Korean Patent Application No. 10-2008-0074970 filed on July 31, 2008, the entire subject matter of which is incorporated herein by reference.

### BACKGROUND OF THE INVENTION

The present invention generally relates to ultrasound systems, and more particularly to an ultrasound system and a method of offering preview pages on a screen.

The ultrasound system has become an important and popular diagnostic tool due to its non-invasive and non-destructive nature. Modem high-performance ultrasound diagnostic systems and techniques are commonly used to produce two- or three-dimensional images of internal features of patients.

The ultrasound system generally uses a probe containing an array of transducer elements to transmit and receive ultrasound signals. The ultrasound system forms an image of internal tissues and organs by electrically exciting transducer elements to generate ultrasound signals that travel into a human body. Echoes reflected from the tissues and organs return to the transducer elements and are converted into electrical signals, which are amplified and processed to produce an ultrasound image.

The ultrasound system can provide a measurement function, which allows a user to measure the size of a specific portion on the ultrasound image. The ultrasound system may further provide a report function for showing a plurality of report pages including the measurement results. The report pages are, for example, very useful to analyze the states and developments of a fetus and the like.

### BRIEF DESCRIPTION OF THE DRAWINGS

- FIG. 1: is a block diagram of an illustrative embodiment of an ultrasound system in accordance with the present invention.
- FIGS. 2 and 3: are schematic diagrams showing a report page and a plurality of preview pages in accordance with the present invention.
- FIG. 4: is a schematic diagram showing a magnified image of one preview page in accordance with the present invention.

### DETAILED DESCRIPTION OF THE INVENTION

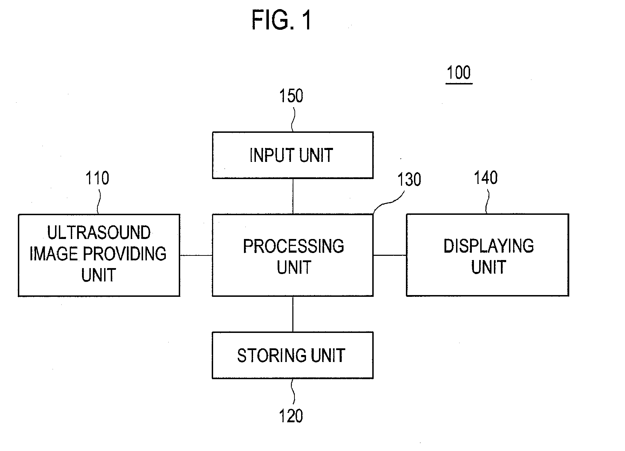

FIG. 1 is a block diagram of an illustrative embodiment of an ultrasound system. The ultrasound system 100 may include an ultrasound image providing unit 110, a storing unit 120, a processing unit 130, a displaying unit 140 and an input unit 150.

The ultrasound image providing unit 110 may provide ultrasound images. The ultrasound image providing unit 110 may be a diagnostic unit that provides the ultrasound images in real time or a storing unit that stores the ultrasound images.

The storing unit 120 may store report pages including information of at least one target object. The target object may be a patient, while the information may be a name, age, gender, ultrasound image, etc. of the patient. The report pages may further include measurements results. The measurement results may be a distance between two points, a non straight-line length between two points, a circumference of a 2-dimensional region, an area of a 2-dimensional region, a volume of a 3-dimensional region in the ultrasound image, etc. The report pages may be formed for every patient as well as for a group of patients.

The report pages may be edited depending on the specific need. For example, when at least two report pages are displayed on the displaying unit 140 in sequence, the user may select at least two parts on the different report pages. The parts may be selected on the report pages in sequence. For example, the user may select a lower part in an earlier report page and an upper part in a latter report page. Also, the user may select one part in a first report page and another part in a fourth report page, which is not right after the first report page. The edited-report page may be also regarded as the report page.

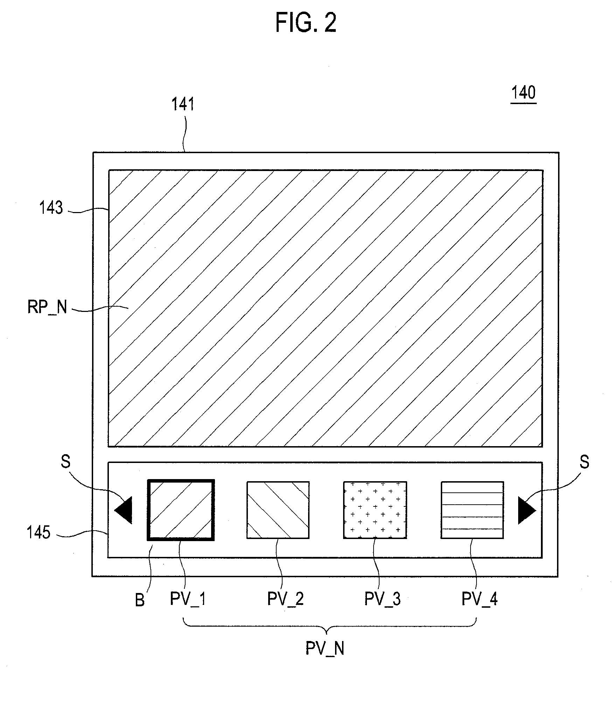

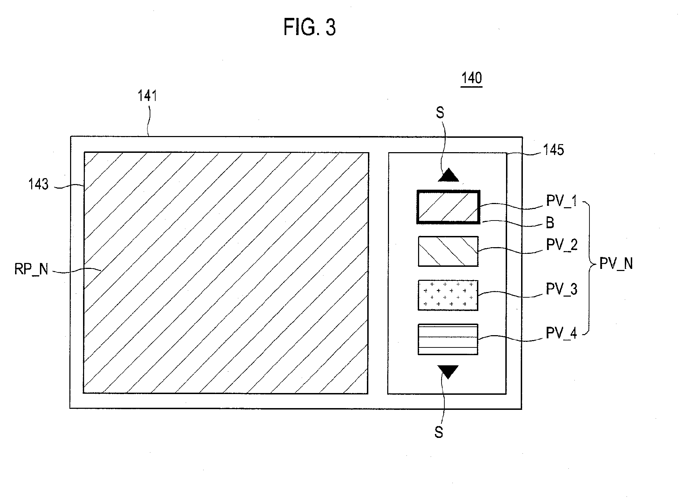

The processing unit 130 may form a plurality of preview pages of the report pages and control display of the preview pages and the report pages. The preview pages and the report pages may have one to one correspondence. The preview pages may be thumbnail images or downsized images of the report pages. The processing unit 130 may retrieve report pages in the storing unit 120 upon receiving a report page retrieving instruction from the input unit 150. The report page retrieving instruction may be a request to display a specific report page or all of the report pages stored in the storing unit 120. In response to the report page retrieving instruction, the processing unit 150 may form the preview pages corresponding to the report pages requested by the user. Under the control of the processing unit 150, one of the requested report pages and a number of preview pages of the requested report pages are displayed on the displaying unit 140. Referring to FIGS. 2 and 3, when the user pushes or drags the scroll buttons S, the processing unit 150 may retrieve other preview pages positioned before or after the preview page PV_1 or PV_4 having been displayed in a preview page region 145 and display the retrieved preview pages on the displaying unit 140. According to the above, the preview pages can be shifted left and right or up and down, sequentially and inverse sequentially.

If the user selects one of the preview pages PV_N by using an enter key (not shown) of the input unit 150 or the cursor C, then the processing unit 150 may display the selected preview page as the report page in the report page region 143 on the screen 141. Further, the processing unit 150 may display the notification mark B on a boundary of the selected preview page PV_1 to show that the selected preview page PV_1 is displayed as the report page RP_N on the screen 141.

The report page region 143 and the preview-page region 145 may be arranged up and down on the screen 141 as shown in FIG. 2, or left and right on the screen 141 as shown in FIG. 3. A lay-out of the report page region 143 and the preview-page region 145 may be changed according to a lay-out changing instruction from the user. Upon receiving the print instruction, the requested report page and/or preview pages may be printed out.

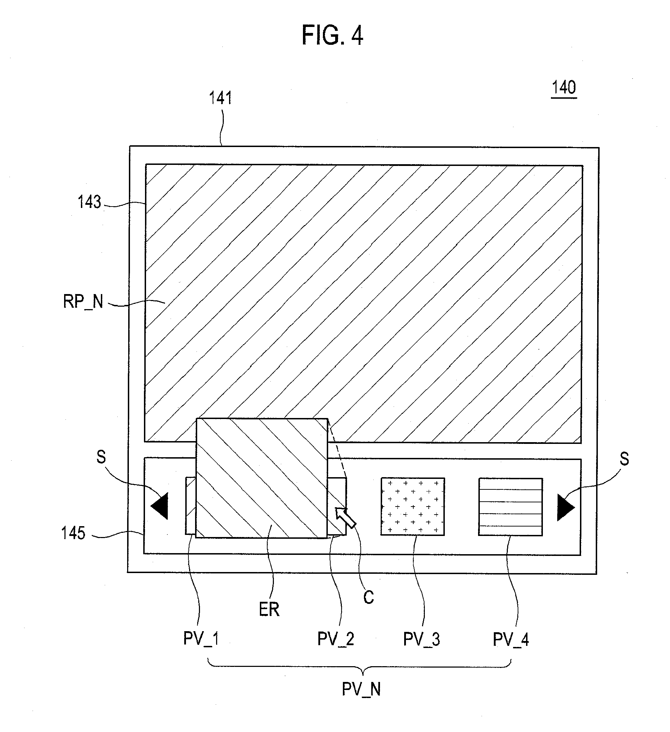

Referring to FIG. 4, if the user moves a cursor on one preview page PV_2 of the preview pages PV_1 to PV_4, then the processing unit 150 may enlarge the one preview page PV_2 to provide a better clear view of the one preview page PV_2.

The displaying unit 140 may display a predetermined number of preview pages PV_1 to PV_4 among all preview pages and one report page RP_N among the report pages when being controlled by the processing unit 130. The displaying unit 140 may display one report page RP_N among the report pages in the report page region 143 and the predetermined number of preview pages PV_1 to PV_4 in the preview page region 145 at a time. Further, the displaying unit 140 may display the scroll buttons S arranged on both sides of the preview page region 145, as shown in FIGS. 2 and 3. Referring to FIGS. 2 to 4, one report page RP_N and a number of preview pages PV_N may be displayed together on the screen 141 of the displaying unit 140. Here, "N" denotes a natural number. A report page RP_N displayed on the screen 141 may be changed whenever the selection instruction from the user is varied.

Referring back to FIG. 1, the input unit 150 may include, but limited to, a control panel, a mouse, a keyboard, etc. The input unit 150 may be configured to allow the user to input instructions such as editing instruction, report page retrieving instruction, preview page selection instruction, lay-out changing instruction and print instruction.

Any reference in this specification to "one embodiment," "an embodiment," "example embodiment," "illustrative embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
a storing unit for storing report pages including an information of at least one target object;
a processing unit for forming a plurality of preview pages of the report pages and controlling a display of the preview pages and the report pages, wherein the preview pages and the report pages correspond one to one;
a displaying unit for displaying a predetermined number of preview pages among the preview pages and one report page among the report pages when being controlled by the processing unit; and
an input unit for receiving a selection instruction of one preview page among the preview pages displayed on the displaying unit from a user, wherein the processing unit is configured to replace the report page displayed on the displaying unit with another report page corresponding to the selected preview page in response to the selection instruction of the preview page.

2. The ultrasound system of Claim 1, wherein the processing unit is configured to enlarge one preview page among the preview pages when a cursor is moved on the one preview page by the user.

3. The ultrasound system of Claim 1, wherein the processing unit is configured to display the preview pages on a bottom of a screen or on a side of the screen.

4. The ultrasound system of Claim 1, wherein the processing unit is configured to change a position of the preview pages on the screen according to a lay-out changing instruction from the user.

5. A method of displaying report pages and preview pages in an ultrasound system, comprising:
receiving an instruction of displaying report pages stored in a storing unit through an input unit within the ultrasound system, wherein the report pages include an information of at least one target object;
forming a plurality of preview pages of the report pages by a processing unit within the ultrasound system, wherein the preview pages and the report pages correspond one to one;
displaying a predetermined number of preview pages among the preview pages and one report page among the report pages at a time on the displaying unit within the ultrasound system; and
receiving a selection instruction of one preview page among the preview pages displayed on the displaying unit through the input unit and replacing the report page displayed on the displaying unit with another report page corresponding to the selected preview page by the processing unit within the ultrasound system.

6. The method of Claim 5, wherein one preview page among the preview pages is enlarged when a cursor is moved on the one preview page by the user.

7. The method of Claim 5, wherein the preview pages are displayed on a bottom of a screen or on a side of the screen.

8. The method of Claim 5, wherein a position of the preview pages on the screen is changed according to a lay-out changing instruction from the user.
